Europäisches Patentamt

European Patent Office

Office européen des brevets

(11) Publication number: **0 638 549 A1**

# EUROPEAN PATENT APPLICATION

(21) Application number: **94110772.4**

(51) Int. Cl.⁶: **C07C 315/06**, C07C 317/22

(22) Date of filing: **12.07.94**

(30) Priority: **16.07.93 JP 199257/93**
 **16.07.93 JP 199258/93**
 **16.07.93 JP 199259/93**

(43) Date of publication of application:
 **15.02.95 Bulletin 95/07**

(84) Designated Contracting States:
 **CH DE FR GB LI**

(71) Applicant: **NICCA CHEMICAL CO., LTD.**
 **23-1, Bunkyo 4-chome**
 **Fukui-shi**
 **Fukui-ken (JP)**

(72) Inventor: **Hosoda, Masaaki**
 **3-18, Oomiya 2-chome**
 **Fukui-shi,**
 **Fukui-ken (JP)**
 Inventor: **Tsubota, Hiroyuki**
 **1208, Nakano 1-chome**
 **Fukui-shi,**
 **Fukui-ken (JP)**
 Inventor: **Tomoda, Yuuichi**
 **37-28, Nakatsuno-cho**
 **Fukui-shi,**
 **Fukui-ken (JP)**
 Inventor: **Makino, Masahiro**
 **31-33, Shimonoda-cho**
 **Sabae-shi,**
 **Fukui-ken (JP)**

(74) Representative: **Türk, Gille, Hrabal, Leifert**
 **Brucknerstrasse 20**
 **D-40593 Düsseldorf (DE)**

(54) A 4,4'-dihydroxydiphenylsulfone monomer composition and a process for production thereof.

(57) A 4,4'-dihydroxydiphenylsulfone monomer composition comprises a salt of an organic acid in the amount corresponding to an amount of the metal component of 10 to 10,000 ppm by weight of the composition. A process for production of a 4,4'-dihydroxydiphenylsulfone monomer composition comprises adding a salt of an organic acid to a solution of 4,4'-dihydroxydiphenylsulfone and drying the mixture to form a composition comprising the salt of an organic acid in the amount corresponding to an amount of the metal component of 10 to 10,000 ppm by weight. Another process for production of a 4,4'-dihydroxydiphenylsulfone monomer composition comprises providing a salt of an organic acid, in the amount corresponding to an amount of the metal component of 10 to 10,000 ppm by weight, to 4,4'-dihydroxydiphenylsulfone in the process of synthesizing a crude 4,4'-dihydroxydiphenylsulfone or in the process of purifying a crude 4,4'-dihydroxydiphenylsulfone. The 4,4'-dihydroxydiphenylsulfone monomer composition is useful as a material in the field of the polymer industry, such as plastics having excellent resistance to yellowing.

## BACKGROUND OF THE INVENTION

### 1. Field of the Invention

The present invention relates to a 4,4'-dihydroxydiphenylsulfone monomer composition which provides a polymer showing no yellowing when the composition is used as the material and a novel process for production thereof. The monomer composition comprises a salt of an organic acid in the amount corresponding to an amount of the metal component in the range of 10 to 10,000 ppm by weight of the composition.

### 2. Description of the Related Art

Monomer compositions containing 4,4'-dihydroxydiphenylsulfone (hereinafter referred to as 4,4'-Compound) are used as materials of plastics having excellent transparency, superior mechanical properties, high heat resistance, good flame retarding property and excellent chemical resistance, such as polyethersulfones and polysulfones, and as materials in other fields of the polymer industry.

However, 4,4'-Compound monomer compositions generally contain impurities, such as 2,4'-dihydroxydiphenylsulfone (hereinafter referred to as 2,4'-Compound), in addition to 4,4'-Compound of the main component. Polymer materials prepared by using a 4,4'-Compound monomer composition containing impurities show yellowing caused by the impurities. No process which can produce a 4,4'-Compound monomer composition showing no yellowing with good yield has heretofore been known.

For production and purification of 4,4'-Compound, processes comprising treatment with various kinds of mixed organic solvent have been proposed (Japanese Patent Publications Showa 51(1976)-36264, Showa 57(1982)-48152, Showa 57(1982)-48153 and Showa 58(1983)-2234). However, these processes have a drawback that they cause environmental problems because they all employ solvents containing halogen.

Another process of two-baths and two-stages which comprises adding an agent for salting out to an aqueous solution of a monometal salt of 4,4'-Compound to salt out the monometal salt, dissolving the separated monometal salt of 4,4'-Compound in water and separating 4,4'-Compound by deposition with addition of an acid to the solution was proposed (Japanese Patent Publications Showa 64(1989)-50855, Showa 64(1989)-50856, Heisei 4(1992)-78609 and Heisei 5(1993)-12344). However, this process has a number of drawbacks that it cannot be applied to a crude product having a lower content of 4,4'-Compound, that an agent for salting out is necessary, and that components responsible for the yellowing cannot be removed. Moreover, it has a serious drawback that, when 4,4'-Compound is prepared by a simple process of deposition with addition of an acid to the aqueous solution, a polymer material showing no yellowing cannot be obtained from 4,4'-Compound prepared. Another process which comprises dissolving 4,4'-Compound and 2,4'-Compound into a basic aqueous solution and recovering 4,4'-Compound by deposition with addition of an acid was proposed (Japanese Patent Publication Heisei 4(1992)-82142). However, recovery of 4,4'-Compound in this process is not high. Purity of the product is not high either even though the recovered product is washed with water to remove trace amounts of impurities. Thus, a process for production of a 4,4'-Compound monomer composition showing no yellowing, in other words, having excellent resistance to yellowing, has been desired in the field of the material for the polymer industry.

## SUMMARY OF THE INVENTION

The present invention accordingly has an object to provide a novel 4,4'-dihydroxydiphenylsulfone monomer composition containing a salt of an organic acid which is useful as a material in the field of the polymer industry, such as plastics having excellent resistance to yellowing, and a process for production thereof.

The present inventors conducted extensive studies to achieve the above object and discovered a surprising fact that a high purity 4,4'-Compound showed excellent resistance to yellowing by the mere presence of a salt of an organic acid together with it. The fact that a 4,4'-Compound composition showing excellent resistance to yellowing could be obtained by simply adding a salt of an organic acid to 4,4'-Compound was entirely unexpected by those who are engaged in the study and production of dihydroxydiphenylsulfone. The present invention which relates to a 4,4'-Compound monomer composition showing excellent resistance to yellowing and various processes for providing a salt of an organic acid to 4,4'-Compound was completed on the basis of the discovery.

Thus, the present invention provides a 4,4'-Compound monomer composition comprising a salt of an organic acid in the amount corresponding to an amount of the metal component in the range of 10 to 10,000 ppm by weight of the composition.

The present invention also provides a process for production of a 4,4'-Compound monomer composition which comprises adding a salt of an organic acid to a solution of a high purity 4,4'-Compound, and drying the mixture to form the composition comprising the salt of an organic acid in the amount corresponding to an amount of the metal component in the range of 10 to 10,000 ppm by weight of the composition.

The present invention also provides a process for production of a 4,4'-Compound monomer composition which comprises providing a salt of an organic acid, in the amount corresponding to an amount of the metal component in the range of 10 to 10,000 ppm by weight of the composition, to 4,4'-Compound in the process of synthesizing a crude 4,4'-Compound or in the process of purifying a crude 4,4'-Compound.

Other and further objects, features and advantages of the invention will appear more fully from the following description.

DETAILED DESCRIPTION OF THE INVENTION

The process of the present invention comprises three modes. In the first mode, a salt of an organic acid is added in the process of production of a crude 4,4'-Compound by the reaction of phenol and sulfuric acid after the acid value of the reaction system as phenolsulfonic acid reaches 25 or lower. In the second mode, a salt of an organic acid is provided to 4,4'-Compound in the process of production of a high purity 4,4'-Compound. For example, a salt of an organic acid is added to the system of the production or formed from an organic acid and a base or an equivalent compound of a base in the system of the production in the process of purification of a crude 4,4'-Compound by a method such as recrystallization, salting out or deposition by addition of an acid. In the third mode, a salt of an organic acid is provided uniformly to a high purity 4,4'-Compound after the high purity compound has been prepared.

In all modes of the present invention, for providing the excellent heat resistance required as a material in the polymer industry to a 4,4'-Compound monomer composition, it is necessary that the 4,4'-Compound monomer composition contains a salt of an organic acid in the amount corresponding to an amount of the metal component in the range of 10 to 10,000 ppm by weight, preferably 10 to 500 ppm by weight, of the composition. When the content is less than the amount corresponding to 10 ppm by weight of the metal component, the 4,4'-Compound monomer composition for the polymer industry showing excellent resistance to yellowing cannot be obtained.

From the view point of providing the resistance to yellowing alone, a salt of an organic acid may be contained in the amount corresponding to an amount of more than 10,000 ppm by weight of the composition. However, when the 4,4'-Compound monomer composition is used as a high quality material in the polymer industry, it is required that an excess amount of the salt be removed by some kind of method.

In contrast to the above description, a 4,4'-Compound monomer composition having excellent resistance to yellowing cannot be obtained when the 4,4'-Compound monomer composition is provided with 10 ppm or more of a metal component in the form of an inorganic salt which is generally used in the process of salting out or generally formed in the process of deposition by addition of an acid, such as sodium sulfate, potassium sulfate, lithium sulfate, magnesium sulfate, calcium sulfate, barium sulfate, sodium chloride, potassium chloride, lithium chloride, magnesium chloride, calcium chloride, barium chloride, sodium nitrate, potassium nitrate, lithium nitrate, magnesium nitrate, calcium nitrate or barium nitrate.

As the salt of an organic acid, a salt formed from a monobasic aliphatic carboxylic acid, a polybasic aliphatic carboxylic acid or an aromatic carboxylic acid and an alkali metal hydroxide or an alkaline earth metal hydroxide can be used.

The organic acid component of the salt of an organic acid is not particularly limited so long as it is an organic acid. As the organic acid, for example, a monobasic aliphatic carboxylic acid, such as formic acid, acetic acid, propionic acid, butyric acid, valeric acid, caproic acid, enanthic acid, caprylic acid, peralgonic acid, capric acid, lauric acid myristic acid, palmitic acid, stearic acid, or the like; a polybasic aliphatic carboxylic acid, such as oxalic acid, malonic acid, succinic acid, glutaric acid, adipic acid, pimellic acid, suberic acid, azelaic acid, sebacic acid, or the like; or an aromatic carboxylic acid, such as benzoic acid, phthalic acid, terephthalic acid, isophthalic acid, trimellitic acid, pyromellitic acid, 4,4'-dicarboxydiphenylsulfone or the like, can preferably be used.

As the base component of the salt of an organic acid, for example, sodium hydroxide, potassium hydroxide, lithium hydroxide, calcium hydroxide, barium hydroxide or the like is preferably used. As the equivalent compound of a base, for example, a carbonate or a hydrogencarbonate of the base component described above is preferably used. A salt of an organic acid formed from the organic acid described above and the base component or the equivalent compound of a base described above is preferably used. When the base component or the equivalent compound of a base described above is used, the salt of an organic acid used in the present invention comprises an alkali metal or an alkaline earth metal, such as sodium potassium, lithium, magnesium, calcium, barium or the like, as the metal component thereof.

Particularly preferable examples of the salt of an organic acid used in the present invention are sodium acetate, potassium acetate, calcium acetate, magnesium acetate, disodium succinate, and disodium isophthalate.

The present inventors further studied the process of purification intensively and discovered that a high purity 4,4'-Compound monomer composition having the resistance to yellowing can be obtained by the following processes. In the first three processes, a salt of an organic acid is provided to the composition in the process of purifying a crude 4,4'-Compound. In the first process, while a crude 4,4'-Compound is dissolved into a mixed solvent of water and a hydrophilic solvent with heating and the solution prepared is cooled for recrystallization, a salt of an organic acid is added to the solution or formed from an organic acid and a base or an equivalent compound of a base in the solution. In the second process, a crude 4,4'-Compound is dissolved into a solvent of an alcohol containing 30 % by weight or less of water and 1 equivalent of a base per 1 mol of dihydroxydiphenylsulfone is added to the solution prepared. A monometal salt of 4,4'-dihydroxydiphenylsulfone is formed and separated by deposition. The separated product is further dissolved in water and then separated by deposition with addition of an acid. In the process of separation by deposition, an organic acid or a combination of an organic acid and a salt of an organic acid is added simultaneously. In the third process, a crude 4,4'-Compound is dissolved in water containing no alcohol by adjusting pH of the solution to 9 or lower using 0.4 to 0.8 equivalent of a base and heating to about 80°C. Then, the product is separated by deposition with adjustment of pH of the solution prepared to 5 to 7 using an organic acid alone or a combination of an organic acid and a mineral acid. Alternatively, an organic acid is added to the solution and then the product is separated by deposition with adjustment of pH to 5 to 7 using a mineral acid. In the fourth process, the 4,4'-Compound monomer composition is produced by adding a salt of an organic acid uniformly to a high purity 4,4'-Compound which has been prepared and purified in advance.

The first process is described in more detail. In this process of the present invention, a crude 4,4'-Compound is dissolved into a mixed solution of water and a hydrophilic solvent by heating with refluxing and the solution thus prepared is then cooled to separate a high purity 4,4'-Compound by recrystallization. While the solution is cooled for the recrystallization, a salt of an organic acid is added to the system or formed from an organic acid and a base or an equivalent compound of a base in the system to provide the salt of an organic acid to the 4,4'-Compound monomer composition. It was discovered that the effect of preventing yellowing is exhibited when the monomer composition comprises the salt of an organic acid in the amount corresponding to an amount of the metal component in the range of 10 to 10,000 ppm by weight, preferably 10 to 500 ppm by weight, of the composition.

The hydrophilic solvent used in the present mode of process is not particularly limited so long as it is a solvent miscible with water. Examples of the hydrophilic solvent include lower aliphatic alcohols, such as methanol, ethanol, n-propanol, isopropanol, n-butanol, isobutanol, sec-butanol, and t-butanol, dimethylformamide, and dimethylsulfoxide. The solvent may be used singly or as a mixture of two or more kinds. Among these solvents, methanol, ethanol, n-propanol and isopropanol are particularly preferable. Amount of the hydrophilic solvent mixed with water and amount of the mixed solvent used for dissolving the crude 4,4'-Compound are suitably decided depending on purity of the crude product, amount of the substances to be treated and kind of the hydrophilic solvent. Temperatures of heating and cooling are also suitably selected depending on the kind of hydrophilic solvent used.

As the salt of an organic acid in the present process, a salt formed from the monobasic aliphatic carboxylic acid, the polybasic aliphatic carboxylic acid, or the aromatic carboxylic acid described above, and a hydrate of an alkali metal or a hydrate of an alkaline earth metal can be used. The preferable examples of the salt of an organic acid include sodium acetate, potassium acetate, calcium acetate, magnesium acetate, disodium succinate, and disodium isophthalate.

As the organic acid, the base and the equivalent compound of a base used in the present process, the organic acid, the base and the equivalent compound of base described above can preferably be used.

The second process is described in more detail in the following. When a monoalkali salt is formed in an aqueous solution from a crude product having purity of 4,4'-Compound of about 70 % or lower by weight, the solution containing the product is separated to an aqueous layer and a layer of a viscous liquid independently of the amount of the solvent used and no crystallization occurs. On the other hand, it was discovered that, when a monometal salt was formed in an alcohol solution containing 30 % by weight or less of water by adding 1 equivalent of an alkali to 1 mol of dihydroxydiphenylsulfone, crystal of a high purity monometal salt of 4,4'-Compound was separated by deposition even when a crude product having purity of 4,4'-Compound of 55 % was used. When the crystal obtained was dissolved in water and a 4,4'-Compound monomer composition was separated by deposition with control of pH using sulfuric acid or hydrochloric acid, a 4,4'-Compound monomer composition having the resistance to yellowing was not obtained. On the other hand, it was surprisingly discovered by the present inventors that, when pH was adjusted using an organic acid in the process of the deposition by addition of an acid or when a salt of an organic acid was added to the solution in the process of the deposition by addition of an acid, the 4,4'-Compound monomer composition for the polymer industry having the resistance to yellowing could be obtained.

Accordingly, in the present process, a crude 4,4'-Compound is dissolved into a solvent of an alcohol containing 30 % by weight or less of water and a base is added to the solution. A monometal salt of 4,4'-Compound is formed and separated by deposition. The separated product is further dissolved in water and then separated by deposition with addition of an inorganic acid. In the process of separation by deposition, pH of the aqueous solution is adjusted to 5 to 7 using an organic acid or using an organic acid and a salt of an organic acid in combination.

In the present process, the crude 4,4'-Compound is first dissolved into a solvent of an alcohol containing 30 % by weight or less of water.

As the alcohol used for the solvent in the present process, any kind of alcohol may be used without particular restriction so long as it has the ability to dissolve 4,4'-Compound. The preferable examples include lower alcohols, such as methanol, ethanol and isopropanol. Isopropanol is particularly preferable because it has low solubility of monoalkali salts of 4,4'-Compound and high solubility of impurities.

Amount of the solvent of an alcohol used in the present process is varied depending on the solubility. For example, when isopropanol is used as the solvent of an alcohol, it is preferably used in an amount of 1.5 to 3.0 parts by weight per 1 part by weight of the crude product of 4,4'-Compound. When the amount of isopropanol used is less than 1.5 parts by weight, the monometal salt of 4,4'-Compound formed has inferior property for filtration to cause lower purity of the product. When the amount is more than 3.0 parts by weight, yield of the formation of the monometal salt of 4,4'-Compound is decreased. The isopropanol solvent may contain some amount of water. When the content of water in isopropanol is more than 30 % by weight, yield of 4,4'-Compound is decreased and efficiency of recovery of the solvent by distillation is also decreased.

Therefore, it is most efficient for increasing the yield and the purity and promoting the recovery of the solvent that isopropanol is used as the aqueous solution having the composition of the azeotropic mixture which contains 15 to 20 % by weight of water.

In the present process, a crude 4,4'-Compound is dissolved in isopropanol by heating to 80 °C and an alkali hydroxide, such as sodium hydroxide or potassium hydroxide, or an aqueous solution thereof is added to the isopropanol solution. Amount of the alkali hydroxide is adjusted to the total of 1 equivalent per 1 mol of the crude 4,4'-Compound and the amount required to neutralize sulfonic acids contained as impurities. When the solution thus prepared is cooled to room temperature, the monoalkali metal salt is separated by deposition without addition of an agent for salting out in a high yield of 93 % or more to obtain a monoalkali salt of 4,4'-Compound having a high purity of 97 % or more.

In the present process, most of colored components of impurities are dissolved in the isopropanol solution and purification in a later stage is facilitated.

As the alkali hydroxide used in the present process, sodium hydroxide is preferable because it has a low solubility in the isopropanol solution of the monoalkali salt of 4,4'-Compound.

In the present process, amount of water used in the stages of dissolving the monosodium salt of 4,4'-Compound and separating the product by deposition with addition of an acid is preferably 2 to 4 parts by weight per 1 part by weight of the monosodium salt of 4,4'-Compound. When the amount is less than 2 parts by weight, concentration of the slurry formed is high and fluidity of the slurry is lacking to cause decrease in purity of the product. When the amount is more than 4 parts by weight, the yield is decreased and the process is economically unfavorable.

As the acid used for the separation by deposition in the present process, sulfuric acid and hydrochloric acid are suitable. Sulfuric acid is preferable because effect of halogen mixed with the composition can be avoided. In an example of separating the product by deposition with addition of sulfuric acid, sulfuric acid is dropped into an aqueous solution of monosodium salt of 4,4'-Compound heated to 80 °C or higher to decrease pH of the solution to 7.5 to 8.0. Then, pH of the solution prepared is adjusted in the range of 5 to 7, preferably 6 to 7, using an organic acid. In another example of separating the product by deposition with addition of sulfuric acid, an alkali metal salt of an organic acid is dissolved in an aqueous solution of monosodium salt of 4,4'-Compound and a high purity 4,4'-Compound is separated by deposition with adjustment of pH to 5 to 7, preferably 6 to 7, using sulfuric acid. In the latter example, the alkali salt of an organic acid is added in an amount of 0.1 to 10 parts by weight per 100 parts by weight of the aqueous solution.

The high purity 4,4'-Compound monomer composition thus obtained has a purity of 99.9 % or more and excellent property as a material for the polymer industry showing no yellowing. In contrast, when pH is adjusted to 6 to 7 using sulfuric acid or hydrochloric acid in the absence of a salt of an organic acid, the 4,4'-Compound monomer composition obtained contains large amounts of 2,4'-Compound and other components to decrease the purity of the product. Resistance to yellowing is decreased as well when it is used as a material for the polymer industry.

The organic acid used for adjusting pH in the present process is not particularly limited but an organic acid having a small number of carbon atom, such as a carboxylic acid having a small number of carbon atoms like formic acid, acetic acid, propionic acid, succinic acid, glutaric acid, adipic acid, benzoic acid or the like, can generally be used. Acetic acid is preferably used.

It is the characteristic of the present process that the substance providing the resistance to yellowing is a sodium salt or a potassium salt of an organic acid independently of the purity of the 4,4'-Compound monomer composition formed and that the salt of an organic acid is effective when it is contained in the amount corresponding to an amount of sodium or potassium in the range of 10 to 10,000 ppm by weight, preferably 10 to 500 ppm, of the composition.

Actually, it was also found that sodium sulfate, potassium sulfate, sodium chloride or potassium chloride which was formed from sulfuric acid or hydrochloric acid added for separation of the product by deposition had no ability to improve the resistance to yellowing at all.

On the other hand, it was found that the resistance to yellowing was remarkably improved when the alkali salt of an organic acid described above in the amount corresponding to an amount of the metal component in the range of 10 to 10,000 ppm by weight, preferably 10 to 500 ppm, was added to a 4,4'-Compound monomer composition having no resistance to yellowing which was obtained from 4,4'-Compound without adjusting pH using an organic acid or to a 4,4'-Compound monomer composition purified by recrystallization and containing no salt of an organic acid.

The third process is described in more detail in the following. It was discovered by the present inventors that, by merely adding 0.4 to 0.8 equivalent of a base per 1 mol of dihydroxydiphenylsulfone to an aqueous solution of a crude 4,4'-Compound to adjust pH to 9 or lower and heating the solution to 80°C or higher, a crude 4,4'-Compound showed a far superior solubility to that of the monoalkali salt of 4,4'-Compound prepared by adding 1 equivalent of a base to 1 mol of dihydroxydiphenylsulfone. The process of production of a 4,4'-Compound monomer composition comprising the purification described in the following has been completed on the basis of this discovery.

According to the present process, a 4,4'-Compound monomer composition having good quality is produced with good efficiency. In the process, a crude 4,4'-Compound is dissolved in a solution containing an equivalent amount or less of an alkali by heating and a high purity 4,4'-Compound having excellent resistance to yellowing and suitable for application in the polymer industry can be obtained from the solution prepared by controlling pH using an organic acid alone or using an organic acid and a mineral acid in combination in a single bath and single step process.

The solubility of dihydroxydiphenylsulfone is extremely decreased when the amount of the base used is less than 0.4 equivalent or more than 0.9 equivalent. This fact shows a discovery overturning the general concept that 4,4'-Compound is dissolved only in a solution containing 1 or more equivalent of alkali. On the basis of this knowledge, a surprising fact was discovered that, from a homogeneous aqueous solution of 4,4'-Compound containing 1 equivalent or less of a base without forming and separating a monoalkali salt or a dialkali salt of 4,4'-Compound, a high purity 4,4'-Compound having excellent resistance to yellowing could be obtained in a single stage by adjusting pH of the solution to 6 to 7 using an organic acid or using an organic acid and a mineral acid in combination. Particularly important points of the fact discovered here are that the 4,4'-Compound monomer composition prepared according to the discovered process contained the salt of an organic acid present as an impurity which acted as the substance providing the resistance to yellowing to the monomer composition independently of the purity of 4,4'-Compound formed while the 4,4'-Compound monomer composition obtained by adjusting pH to 6 to 9 using a mineral acid alone had no resistance to yellowing required as a material for the polymer industry, and that the resistance to yellowing was surprisingly improved when the salt of an organic acid was contained in the amount corresponding to an amount of a metal of 10 ppm or more. The present process was completed on the basis of these discoveries.

Accordingly, the present process of production of a 4,4'-Compound monomer composition comprises a process of purification of a crude 4,4'-Compound in an aqueous medium in which process the crude product is dissolved in water using 0.4 to 0.8 equivalent of a base per 1 mol of 4,4'-Compound and a high purity 4,4'-Compound is separated by deposition with adjustment of pH of the solution prepared to 6 to 7 using an organic acid alone or using an organic acid and a mineral acid in combination, and another process of purification of a crude 4,4'-Compound monomer composition in an aqueous medium in which process the crude product is dissolved in water by adjusting pH to about 9 using 0.4 to 0.8 equivalent of a base per 1 mol of 4,4'-Compound, a salt of an organic acid is added to the aqueous solution thus prepared and a high purity 4,4'-Compound is separated by deposition with adjustment of pH of the solution to 6 to 7 using a mineral acid.

In the present process, the crude 4,4'-Compound and the base are dissolved in water by heating at first. pH of the solution thus prepared is about 9. Amount of water is preferably 1.5 parts by weight or more per 1 part by weight of the crude 4,4'-Compound. When the amount of water is less than 1.5 parts by weight, property for filtering the separated crystal of the high purity 4,4'-Compound is inferior and purity of the product is decreased. Amount of the base used is 0.4 to 0.8 equivalent, preferably 0.4 to 0.6 equivalent, more preferably 0.45 to 0.55 equivalent, per 1 mol of the crude 4,4'-Compound.

As the base in the present process, sodium hydroxide, potassium hydroxide, lithium hydroxide, magnesium hydroxide, calcium hydroxide, or barium hydroxide is preferably used. Carbonates corresponding to these compounds can also be preferably used as the equivalent compounds. While temperature of the solution prepared above is kept at 80°C or higher, pH of the solution is adjusted to a specified value using an organic acid or using an organic acid and a mineral acid in combination.

In another mode of adjusting pH in the present process, pH can be adjusted to a specified value by adding a mineral acid after the salt of an organic acid is dissolved into the system.

In the present process, pH of the solution is adjusted in the range of 6 to 7, preferably 6.4 to 6.8, using an acid. When pH is adjusted in this range, a 4,4'-Compound monomer composition having the purity of 99.8 % or more can be obtained with the yield of 83 % or more without decrease in the yield.

As the mineral acid in the present process, sulfuric acid, hydrochloric acid, nitric acid or phosphoric acid is used.

As the salt of an organic acid in the present process, the salt obtained from an organic acid and a base described above can be used. Amount of the added salt of an organic acid is 0.1 to 10 parts by weight, preferably 0.5 to 5 parts by weight, based on 100 parts by weight of a 35 % aqueous solution of the crude 4,4'-Compound.

To summarize the advantages obtained by the invention, the high purity 4,4'-Compound produced according to the process of the present invention has the excellent resistance to yellowing because it contains a small amount of a salt of an organic acid and can be favorably used as a material in the polymer industry.

The invention will be understood more readily with reference to the following examples; however, these examples are intended to illustrate the invention and are not to be construed to limit the scope of the invention.

Measurements in the examples were conducted according to the methods described in the following. The material used in the examples was synthesized according to the method also described in the following.

(1) Measurement of purity

Contents of 4,4'-Compound or monosodium salt thereof and 2,4'-Compound or monosodium salt thereof were measured using the high performance liquid chromatography.

(2) Measurement of heat resistance (test of resistance to yellowing)

Change after heat treatment at 280°C for 10 minutes in the air was evaluated by the APHA method (Hazen No.).

(3) Synthesis of the crude 4,4'-Compound used in the examples

Using 720 parts by weight of phenol and 300 parts by weight of sulfuric acid, dehydration reaction was conducted under a reduced pressure without a solvent. The reaction was finished when the acid value reached 10 or less to obtain a crude 4,4'-Compound (content of 4,4'-Compound, 94.0 %; content of 2,4'-Compound, 2.0 %; content of other components, 4.0 %) (BPS-1, a trade name).

Example 1

Into a reactor, 300 g of the crude 4,4'-Compound prepared above (BPS-1, a trade name) and 450 g of methanol containing 30 % of water were charged and the crude product was dissolved by heating under refluxing. The solution was then cooled to room temperature. Crystalline deposit was separated by filtration with suction and dried to obtain the product in the yield of 85 %. The product had the composition of 99.8 % of 4,4'-Compound, 0.14 % of 2,4'-Compound and 0.06 % of other components. To 100 g of the powder of 4,4'-Compound thus obtained, 70 mg of sodium acetate was added and mixed in a blender. After the test of resistance to yellowing, the product had the hue of ≤ 350 according to APHA.

Example 2

Into a reactor, 300 g of the crude 4,4'-Compound prepared above (BPS-1, a trade name) and 450 g of methanol containing 30 % of water were charged and the crude product was dissolved by heating under refluxing. Then, 6 g of sodium acetate was added to the solution to form a homogeneous solution and the solution prepared was cooled to room temperature. Crystalline deposit was separated by filtration with suction and dried to obtain the product in the yield of 85 %. The product had the composition of 99.8 % of 4,4'-Compound, 0.14 % of 2,4'-Compound and 0.06 % of other components and contained 20 ppm of sodium metal. After the test of resistance to yellowing, the product had the hue of ≤ 350 according to APHA.

Example 3

By the same process as that in Example 1 except that 4 g of disodium succinate was added in place of sodium acetate, a product having the composition of 99.8 % of 4,4'-Compound, 0.14 % of 2,4'-Compound and 0.06 % of other components and containing 17 ppm of sodium metal was obtained. After the test of resistance to yellowing, the product had the hue of ≤ 350 according to APHA.

Example 4

By the same process as that in Example 1 except that 4 g of disodium isophthalate was added in place of sodium acetate, a product having the composition of 99.8 % of 4,4'-Compound, 0.14 % of 2,4'-Compound and 0.06 % of other components and containing 12 ppm of sodium metal was obtained. After the test of resistance to yellowing, the product had the hue of ≤ 350 according to APHA.

Example 5

Into a reactor, 300 g of the crude 4,4'-Compound prepared above (BPS-1, a trade name) and 450 g of an 85 % aqueous solution of isopropanol were charged and the crude product was dissolved by heating under refluxing. Then, 49.0 g of sodium hydroxide was added to the solution to form monosodium salt of 4,4'-Compound. The solution prepared was cooled to room temperature to separate crystal by deposition. After the crystal was separated, it was dissolved in 450 g of water by heating. To the solution obtained, 37.5 g of sulfuric acid was added to adjust pH of the solution to 7.5. Then, 15.0 g of acetic acid was added to the solution to adjust pH of the solution to 6.7. The solution was then cooled to room temperature and crystalline deposit was separated by filtration with suction and dried to obtain the product in the yield of 85 %. The product had the composition of 99.96 % of 4,4'-Compound, 0.04 % of 2,4'-Compound and 0.0 % of other components and contained 350 ppm of sodium metal. In the total sodium metal contained, content of sodium metal as the acetate was 25 ppm. After the test of resistance to yellowing, the product had the hue of ≤ 350 according to APHA.

Example 6

Using 720 parts by weight of phenol and 300 parts by weight of sulfuric acid, dehydration reaction was conducted under a reduced pressure without a solvent. When the acid value reached 20, 15 g of sodium acetate was added and the reaction was further continued. When the acid value reached 10, the reaction was finished and a crude 4,4'-Compound (content of 4,4'-Compound, 94.0 %; content of 2,4'-Compound, 2.0 %; content of other components, 4.0 %), was obtained. Into a reactor, 300 g of the crude 4,4'-Compound thus obtained and methanol containing 30 % of water were charged and the crude product was dissolved by heating under refluxing. The solution was then cooled to room temperature and crystalline deposit was separated by filtration with suction and dried to obtain the product in the yield of 85 %. The product had the composition of 99.6 % of 4,4'-Compound, 0.24 % of 2,4'-Compound and 0.16 % of other components and contained 25 ppm of sodium metal. After the test of resistance to yellowing, the product had the hue of ≦ 350 according to APHA.

Comparative Example 1

Into a reactor, 300 g of the crude 4,4'-Compound obtained above (in Example 6) and 450 g of methanol containing 30 % of water were charged and the crude product was dissolved by heating under refluxing. The solution was then cooled to room temperature and crystalline deposit was separated by filtration with suction and dried to obtain the product in the yield of 85 %. The product had the composition of 99.8 % of 4,4'-Compound, 0.14 % of 2,4'-Compound and 0.06 % of other components. The process conducted here was different from those of Examples in that no salt of an organic acid was added.

Hue after the test of resistance to yellowing could not be measured according to APHA. The product turned into black tar.

Comparative Example 2

Into 200 g of methanol containing 30 % of water, 100 g of the crystal of 4,4'-Compound obtained in Example 5 was dissolved by heating and the solution was then cooled to room temperature. Crystalline deposit was separated by filtration with suction and dried to obtain the product in the yield of 85 %. The product had the composition of 99.91 % of 4,4'-Compound, 0.04 % of 2,4'-Compound and 0.05 % of other components and contained 5 ppm of sodium metal. Hue after the test of resistance to yellowing could not be measured according to APHA. The product turned into black tar.

Reference Example 1

Into a reactor, 300 g of the crude 4,4'-Compound described above (BPS-1, a trade name) and 450 g of an 85 % aqueous solution of isopropanol (containing 15 % by weight of water) were charged and the crude product was dissolved by heating at 80°C. To this solution, 102 g of a 48 % aqueous solution of sodium hydroxide was dropped. The solution was then cooled to room temperature. Crystalline deposit of monosodium salt of 4,4'-Compound was separated by filtration with suction and dried to obtain the product in the yield of 95 %. The product had the composition of 97.55 % of 4,4'-Compound, 0.46 % of 2,4'-Compound and 1.99 % of other components.

Reference Example 2

The crude 4,4'-Compound described above (BPS-1, a trade name) was recrystallized from an alcohol solution and a residue (content of 4,4'-Compound, 72.5 %; content of 2,4'-Compound, 12.0 %; content of other components, 15.5 %) was obtained by removing volatile components from the solution left after the recrystallization. Into a reactor, 300 g of the residue and 450 g of an 85 % aqueous solution of isopropanol were charged and the residue was dissolved by heating to 80°C. To the solution thus obtained, 102 g of a 48 % aqueous solution of sodium hydroxide was dropped and the solution was then cooled to room temperature.

The crystalline deposit of monosodium salt of 4,4'-Compound was separated by filtration with suction and dried to obtain the product in the yield of 65 %. The product had the composition of 97.05 % of 4,4'-Compound, 0.48 % of 2,4'-Compound and 2.47 % of other components.

Example 7

Into a reactor, 100 g of the crystal obtained in Reference Example 1 and 300 g of water were charged and the crystal was dissolved by heating. To the solution, sulfuric acid was dropped at 90°C until pH of the solution decreased to 7.5 and then pH of the solution was adjusted to 6.5 using acetic acid. The solution was cooled to room temperature and the crystalline deposit of a 4,4'-Compound monomer composition was separated by filtration with suction and dried. Yield of the product based on the amount of the monosodium salt of 4,4'-Compound was 95 %. The product had the composition of 99.95 % of 4,4'-Compound, 0.05 % of 2,4'-Compound and 0 % of other components and contained 25 ppm of sodium metal. After the test of resistance to yellowing, the product had the hue of ≤ 350 according to APHA.

Example 8

Into a reactor, 100 g of the crystal obtained in Reference Example 2, 300 g of water and 0.5 g of sodium acetate were charged and the components were dissolved by heating. pH of the solution prepared was adjusted to 6.5 by dropping sulfuric acid to the solution at 90°C. Then, the solution was cooled to room temperature and the crystalline deposit of a 4,4'-Compound monomer composition was separated by filtration with suction and dried. Yield of the product based on the amount of the monosodium salt of 4,4'-Compound was 94 %. The product had the composition of 99.94 % of 4,4'-Compound, 0.06 % of 2,4'-Compound and 0 % of other components and contained 11 ppm of sodium metal. After the test of resistance to yellowing, the product had the hue of ≤ 350 according to APHA.

Comparative Example 3

Into a reactor, 100 g of the crystal obtained in Reference Example 1 and 300 g of water were charged and the crystal was dissolved by heating. Sulfuric acid was dropped to the solution prepared at 90°C and pH of the solution was decreased to 7.1. The solution was then cooled to room temperature and the crystalline deposit was separated by filtration with suction and dried. Yield of the product was 92 % based on the monosodium salt of 4,4'-Compound. The product had the composition of 99.57 % of 4,4'-Compound, 0.39 % of 2,4'-Compound and 0.04 % of other components and contained 5 ppm or less of sodium metal. Hue after the test of resistance to yellowing could not be measured according to APHA because the product turned into tar.

Comparative Example 4

The crystal obtained in Example 7 in an amount of 80 g was dissolved in 300 milliliter of a 60 % aqueous solution of methanol (containing 40 % by weight of water) by heating. The solution prepared was cooled to room temperature. Inorganic salts and salts of acetic acid were removed by recrystallization and content of sodium was adjusted to 5 ppm or lower. The product had the composition of 99.85 % of 4,4'-Compound, 0.03 % of 2,4'-Compound and 0.12 % of other components and contained 4 ppm or less of sodium metal. Hue after the test of resistance to yellowing could not be measured according to APHA because the product turned into tar.

Example 9

The crystal obtained in Comparative Example 4 in an amount of 50 g was dissolved in 150 g of a 60 % aqueous solution of methanol containing 0.3 % by weight of sodium acetate. The solution prepared was cooled for recrystallization and a 4,4'-Compound monomer composition containing sodium acetate in the amount corresponding to 15 ppm of sodium metal was obtained. Resistance to yellowing of the 4,4'-Compound monomer composition thus obtained was evaluated. The product had the composition of 99.85 % of 4,4'-Compound, 0.03 % of 2,4'-Compound and 0.12 % of other components and contained 15 ppm of sodium metal. After the test of resistance to yellowing, the product had the hue of ≤ 350 according to APHA.

Comparative Example 5

The crystal obtained in Comparative Example 4 in an amount of 30 g was dissolved into 120 g of a 60 % aqueous solution of methanol containing 1.0 % by weight of sodium sulfate. The solution prepared was cooled for recrystallization and a 4,4'-Compound monomer composition containing sodium sulfate in the amount corresponding to 30 ppm of sodium metal was obtained. Resistance to yellowing of the product thus obtained was evaluated. The product had the composition of 99.85 % of 4,4'-Compound, 0.03 % of 2,4'-Compound and 0.12 % of other components and contained 30 ppm of sodium metal. Hue after the test of resistance to yellowing could not be measured by APHA because the product turned into tar.

Example 10

Into a reactor, 300 g of a crude 4,4'-Compound (BPS- 1, a trade name), 450 g of water and 24.8 g of sodium hydroxide were charged and the components were dissolved by heating to 80°C or higher. After adjusting pH of the solution prepared to 6.7 by adding 37 g of sulfuric acid and 10 g of acetic acid, the solution was cooled to room temperature. Crystalline deposit of a 4,4'-Compound monomer composition was separated by filtration with suction and dried to obtain the product in the yield of 85 %. The product had the composition of 99.8 % of 4,4'-Compound, 0.1 % of 2,4'-Compound and 0.1 % of other components and contained sodium acetate the amount corresponding to 15 ppm of sodium metal. After the test of resistance to yellowing, the product had the hue of ≦ 350 according to APHA.

Example 11

Into a reactor, 300 g of a crude 4,4'-Compound (BPS-1, a trade name), 450 g of water, 24.8 g of sodium hydroxide and 8 g of disodium isophthalate were charged and the components were dissolved by heating to 80°C or higher. After adjusting pH of the solution prepared to 6.5 by adding 42 g of sulfuric acid, the solution was cooled to room temperature. Crystalline deposit of a 4,4'-Compound monomer composition was separated by filtration with suction and dried to obtain the product in the yield of 87 %. The product had the composition of 99.7 % of 4,4'-Compound, 0.2 % of 2,4'-Compound and 0.1 % of other components and contained disodium isophthalate in the amount corresponding to 15 ppm of sodium metal. After the test of resistance to yellowing, the product had the hue of ≦ 350 according to APHA.

Comparative Example 6

A high purity 4,4'-Compound was obtained by the same process as that in Example 10 except that pH of the solution was adjusted to 6.7 without using acetic acid.

The product had the composition of 99.8 % of 4,4'-Compound, 0.1 % of 2,4'-Compound and 0.1 % of other components. Hue after the test of resistance to yellowing could not be measured by APHA because the product turned into tar.

The results of the evaluation are summarized in Table 1.

Table 1

| | 4,4'-Compound (%) | 2,4'-Compound (%) | other components (%) | concentration of sodium[1] | heat resistance (APHA) |
|---|---|---|---|---|---|
| Example 1 | 99.8 | 0.14 | 0.06 | 196 | ≤350 |
| Example 2 | 99.8 | 0.14 | 0.06 | 20 | ≤350 |
| Example 3 | 99.8 | 0.14 | 0.06 | 17 [2] | ≤350 |
| Example 4 | 99.8 | 0.14 | 0.06 | 12 [3] | ≤350 |
| Example 5 | 99.96 | 0.04 | 0.0 | 25 | ≤350 |
| Example 6 | 99.6 | 0.24 | 0.16 | 25 | ≤350 |
| Comparative Example 1 | 99.8 | 0.14 | 0.06 | – | tar formed |
| Comparative Example 2 | 99.91 | 0.04 | 0.05 | 5 | tar formed |
| Reference Example 1 | 97.55 | 0.46 | 1.99 | 0 | – |
| Reference Example 2 | 97.05 | 0.48 | 2.47 | 0 | – |
| Example 7 | 99.95 | 0.05 | 0 | 25 | ≤350 |
| Example 8 | 99.94 | 0.06 | 0 | 11 | ≤350 |
| Comparative Example 3 | 99.57 | 0.39 | 0.04 | ≤5 | tar formed |
| Comparative Example 4 | 99.85 | 0.03 | 0.12 | 4 | tar formed |
| Example 9 | 99.85 | 0.03 | 0.12 | 15 | ≤350 |
| Comparative Example 5 | 99.85 | 0.03 | 0.12 | 30 [4] | tar formed |
| Example 10 | 99.8 | 0.1 | 0.1 | 15 | ≤350 |
| Example 11 | 99.7 | 0.2 | 0.1 | 15 [3] | ≤350 |
| Comparative Example 6 | 99.8 | 0.1 | 0.1 | 15 [4] | tar formed |

Notes: 1) Concentration in ppm of sodium metal contained in the crystal in the form of sodium acetate.

2) Concentration in ppm of sodium metal contained in the crystal in the form of

disodium succinate.

3) Concentration in ppm of sodium metal contained in the crystal in the form of disodium isophthalate.

4) Concentration in ppm of sodium metal contained in the crystal in the form of sodium sulfate.

While the invention has been particularly shown and described with reference to preferred embodiments thereof, it will be understood by those skilled in the art that the foregoing and other changes in form and details can be made therein without departing from the spirit and scope of the invention.

**Claims**

1. A 4,4'-dihydroxydiphenylsulfone monomer composition comprising a salt of an organic acid in the amount corresponding to an amount of the metal component in the range of 10 to 10,000 ppm by weight of the composition.

2. A 4,4'-dihydroxydiphenylsulfone monomer composition as claimed in Claim 1, wherein the salt of an organic acid is comprised in the amount corresponding to an amount of the metal component in the range of 10 to 500 ppm by weight of the composition.

3. A 4,4'-dihydroxydiphenylsulfone monomer composition as claimed in Claim 1 or 2, wherein the salt of an organic acid is formed from a compound selected from the group consisting of monobasic aliphatic carboxylic acids, polybasic aliphatic carboxylic acids and aromatic carboxylic acids, and another compound selected from the group consisting of hydroxides of alkali metals and hydroxides of alkaline earth metals.

4. A process for production of a 4,4'-dihydroxydiphenylsulfone monomer composition which comprises adding a salt of an organic acid to a solution of a high purity 4,4'-dihydroxydiphenylsulfone and drying the mixture to form the composition comprising the salt' of an organic acid in the amount corresponding to an amount of the metal component in the range of 10 to 10,000 ppm by weight of the composition.

5. A process for production of a 4,4'-dihydroxydiphenylsulfone monomer composition which comprises providing a salt of an organic acid, in the amount corresponding to an amount of the metal component in the range of 10 to 10,000 ppm by weight of the composition, to 4,4'-dihydroxydiphenylsulfone in the process of synthesizing a crude 4,4'-dihydroxydiphenylsulfone or in the process of purifying a crude 4,4'-dihydroxydiphenylsulfone.

6. A process for production of a 4,4'-dihydroxydiphenylsulfone monomer composition as claimed in Claim 4 or 5, wherein the salt of an organic acid is formed by adding an organic acid and a base successively to the solution of a high purity 4,4'-dihydroxydiphenylsulfone, the system of synthesizing a crude 4,4'-dihydroxydiphenylsulfone or the system of purifying a crude 4,4'-dihydroxydiphenylsulfone.

7. A process for production of a 4,4'-dihydroxydiphenylsulfone monomer composition as claimed in Claim 4 or 5, wherein the salt of an organic acid is formed from a compound selected from the group consisting of monobasic aliphatic carboxylic acids, polybasic aliphatic carboxylic acids and aromatic carboxylic acids, and another compound selected from the group consisting of hydroxides of alkali metals and hydroxides of alkaline earth metals.

8. A process for production of a 4,4'-dihydroxydiphenylsulfone monomer composition as claimed in Claim 5, wherein, in the process of synthesizing a crude 4,4'-dihydroxydiphenylsulfone, the salt of an organic acid is added to the reaction system for producing the crude 4,4'-dihydroxydiphenylsulfone from phenol and sulfuric acid in the amount corresponding to an amount of the metal component in the range of 10 to 10,000 ppm by weight of the product.

**9.** A process for production of a 4,4'-dihydroxydiphenylsulfone monomer composition as claimed in Claim 5, wherein, in the process of purifying a crude 4,4'-dihydroxydiphenylsulfone which comprises dissolving the crude 4,4'-dihydroxydiphenylsulfone in a mixed solvent of water and a hydrophilic solvent by heating and then cooling the solution prepared for recrystallization, the salt of an organic acid is added to the solution or formed from an organic acid and a base or an equivalent compound of a base in the solution.

**10.** A process for production of a 4,4'-dihydroxydiphenylsulfone monomer composition as claimed in Claim 5, wherein the process of purifying a crude 4,4'-dihydroxydiphenylsulfone comprises dissolving the crude 4,4'-dihydroxydiphenylsulfone in a solvent of an alcohol containing 30 % by weight or less of water, separating a high purity monometal salt of 4,4'-dihydroxydiphenylsulfone by deposition with addition of an aqueous solution of a monoacidic base to the solution prepared, dissolving the high purity monometal salt in water to prepare an aqueous solution and separating a high purity 4,4'-dihydroxydiphenylsulfone by deposition with addition of an inorganic acid from the aqueous solution prepared while pH of the aqueous solution is adjusted to 5 to 7 using an organic acid or a salt of an organic acid in combination with the inorganic acid.

**11.** A process for production of a 4,4'-dihydroxydiphenylsulfone monomer composition as claimed in Claim 5, wherein the process of purifying a crude 4,4'-dihydroxydiphenylsulfone comprises dissolving the crude 4,4'-dihydroxydiphenylsulfone into water of pH of 9 or lower using a base in an amount of 0.4 to 0.8 equivalent per 1 mol of dihydroxydiphenylsulfone and separating a high purity 4,4'-dihydroxydiphenylsulfone by deposition with adjustment of pH of the aqueous solution to 5 to 7 using an organic acid alone or using an organic acid and a mineral acid in combination.

**12.** A process for production of a 4,4'-dihydroxydiphenylsulfone monomer composition as claimed in Claim 5, wherein the process of purifying a crude 4,4'-dihydroxydiphenylsulfone comprises dissolving the crude 4,4'-dihydroxydiphenylsulfone in water of pH of 9 or lower using a base in an amount of 0.4 to 0.8 equivalent per 1 mol of dihydroxydiphenylsulfone, adding the salt of an organic acid to the aqueous solution prepared and then separating a high purity 4,4'-dihydroxydiphenylsulfone by deposition with adjustment of pH of the aqueous solution to 5 to 7 with a mineral acid.

**13.** A process for production of a 4,4'-dihydroxydiphenylsulfone monomer composition as claimed in Claim 10, wherein the alcohol is isopropanol.

**14.** A process for production of a 4,4'-dihydroxydiphenylsulfone monomer composition as claimed in Claim 10, wherein the monoacidic base is sodium hydroxide or potassium hydroxide.

**15.** A process for production of a 4,4'-dihydroxydiphenylsulfone monomer composition as claimed in Claim 9, 10 or 11, wherein the organic acid is at least one or more compounds selected from the group consisting of aliphatic carboxylic acids and aromatic carboxylic acids.

**16.** A process for production of a 4,4'-dihydroxydiphenylsulfone monomer composition as claimed in Claim 9, 11 or 12, wherein the base is a compound selected from the group consisting of sodium hydroxide, potassium hydroxide, lithium hydroxide, magnesium hydroxide, calcium hydroxide, and barium hydroxide.

**17.** A process for production of a 4,4'-dihydroxydiphenylsulfone monomer composition as claimed in Claim 9, wherein the equivalent compound of a base is a compound selected from the group consisting of sodium carbonate, sodium hydrogencarbonate, potassium carbonate, potassium hydrogencarbonate, lithium carbonate, lithium hydrogencarbonate, magnesium carbonate, calcium carbonate, and barium carbonate.

**18.** A process for production of a 4,4'-dihydroxydiphenylsulfone monomer composition as claimed in Claim 11 or 12, wherein the mineral acid is a compound selected from the group consisting of sulfuric acid, hydrochloric acid, nitric acid and phosphoric acid.

**19.** A process for production of a 4,4'-dihydroxydiphenylsulfone monomer composition as claimed in Claim 8, 9, 10 or 12, wherein the salt of an organic acid is a compound selected from the group consisting of sodium acetate, potassium acetate, calcium acetate, magnesium acetate, disodium succinate, and disodium isophthalate.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EP-A-0 220 855 (AMOCO CO) <br><br> * column 5, lines 7-32; column 6, lines 15-18; examples * | 1,3,5-7, 9-11, 13-18 | C07C315/06 <br> C07C317/22 |
| D | & JP-B-4 082 142 (...) <br> --- | | |
| A | DE-A-15 43 637 (GENERAL ELECTRIC CO) <br><br> * example 1, in particular page 6, lines 20-30 * <br> ----- | 1,3,5-7, 9,11,15, 16 | |

TECHNICAL FIELDS
SEARCHED      (Int.Cl.6)

C07C

The present search report has been drawn up for all claims

| Place of search | Date of completion of the search | Examiner |
|---|---|---|
| BERLIN | 17 November 1994 | Van Amsterdam, L |

EPO FORM 1503 03.82 (P04C01)